# EUROPÄISCHE PATENTSCHRIFT

(11) **EP 2 877 098 B1**
(45) Veröffentlichungstag und Bekanntmachung des Hinweises auf die Patenterteilung: **31.08.2016**
(21) Anmeldenummer: 13724600.5
(22) Anmeldetag: 24.05.2013
(51) Int. Cl.: A61B 17/00, A61B 17/14, A61B 17/16

(54) **HALTER FÜR EIN MEDIZINISCHES, INSBESONDERE CHIRURGISCHES INSTRUMENT**
HOLDER FOR A MEDICAL INSTRUMENT, IN PARTICULAR A SURGICAL INSTRUMENT
SUPPORT POUR INSTRUMENT MÉDICAL, NOTAMMENT CHIRURGICAL

(30) Priorität: 24.07.2012 EP 12177714
(43) Veröffentlichungstag der Anmeldung: 03.06.2015
(73) Patentinhaber: Waldemar Link GmbH & Co. KG, 22339 Hamburg (DE)
(72) Erfinder: DMUSCHEWSKY, Klaus, 22397 Hamburg (DE); BALZARINI, Amos, 22844 Norderstedt (DE); FREISBERG, Dörte, 20249 Hamburg (DE)
(74) Vertreter: Raffay & Fleck
(86) Internationale Anmeldenummer: PCT/EP2013/060736
(87) Internationale Veröffentlichungsnummer: WO 2014/016011

(56) Entgegenhaltungen:
- EP-A1- 1 943 966
- DE-A1- 10 357 104
- US-A- 2 784 987
- US-A- 6 139 214

## Beschreibung

### Technisches Gebiet

Die vorliegende Erfindung betrifft einen Halter für ein medizinisches, insbesondere chirurgisches Instrument mit einer Schnellkupplung für die Aufnahme eines einen Hinterschnitt aufweisenden Anschlussendes eines Instrumentenschafts des medizinischen Instrumentes, wobei die Schnellkupplung ein erstes Kupplungselement mit einer ersten axialen Durchgangsöffnung für das Anschlussende aufweist.

Ein solcher Halter dient der zumindest axial gehaltenen Festlegung eines medizinischen Instrumentes für dessen Einsatz und Gebrauch. Die axiale Festlegung kann dabei eine solche sein, die ein axiales Spiel zwischen dem Halter und dem Instrument ermöglicht, das Instrument jedoch sicher an dem Halter festlegt, ohne dass es sich in axialer Richtung von dem Halter etwa unbeabsichtigt löst. In vielen Fällen müssen mit dem Halter - sei es über einen motorischen Antrieb erzeugte oder sei es manuell aufgebrachte - Kräfte nicht nur in axialer Richtung, sondern auch für einen rotatorischen Antrieb des medizinischen Instrumentes übertragen werden. In solchen Fällen sind bei entsprechenden Haltern zusätzlich auch Mittel für eine verdrehfeste Aufnahme des medizinischen Instrumentes und Verbindung desselben mit dem Halter vorgesehen.

Ein medizinisches Instrument im Sinne der vorliegenden Beschreibung und Erfindung kann insbesondere ein medizinisch eingesetztes Werkzeug, wie ein Bohrer, eine Fräse, eine Raspel, eine Ahle, eine Säge oder dgl. sein. Unter einem chirurgischen Instrument sind hier aber auch Bestandteile von Implantaten oder vollständige Implantate zu verstehen, seien dies Implantate als Bestandteile von Prothesen, insbesondere auch Endoprothesen, oder sonstige mit einem Halter für deren Handhabung beim Setzen, Entfernen oder Versetzen oder sonstigen Schritten zu verbindende Implantate.

### Stand der Technik

Ein gattungsgemäßer Halter ist beispielsweise in der US 6,139,214 offenbart.Dort wird ein mit einem Hinterschnitt ausgebildetes Schaftende eines chirurgischen Werkzeuges in einer Aufnahme festgelegt, indem es in eine axiale Aufnahmeborung eingesetzt und dort von einem einen axialen Durchtritt für das Schaftenede aufweisenden, quer zu der axialen Erstreckung verschiebbaren, den Hinterschnitt hintergreifenden Riegelstück gehalten wird. Das Riegelstück ist als separates Teil ausgebildet und von einer gesonderten Feder in die Riegelposition vorgespannt. Eine ähnliche Gestaltung wird durch die DE 103 57 104 A1 offenbart, bei der ein Instrumenteneinsatz mit einem Hinterschnitt an einem freien Ende eines Anschlussstücks mittels als elastische Zungen ausgebildeten Rastelementen in einer Aufnahme einer Instrumentenhandhabe lösbar festgelegt wird. Auch die US 2,784,987 zeigt eine nach diesem Prinzip arbeitende Konstruktion, hier für das Verbinden von rohrförmigen Elementen. Schließlich zeigt die DE 60 2004 001 063 T2 in Fig. 1 als Beispiel eines medizinischen Instrumentes eine Reibahle gezeigt, die an einem Anschlussende ihres Instrumentenschafts, dort mit der Bezugsziffer 78 bezeichnet, eine als Hudson-Verbinder oder auch Trinkl-Adapter bezeichnete Anschlusskonfiguration aufweist, die in eine Schnellkupplung eines Halters zum dortigen Festlegen eingeführt wird, welcher Halter nach Art eines Spannfutters mit Spannkrallen, die durch Aufschrauben einer Spannmuffe in einen Hinterschnitt des Anschlussendes des Instrumentenschafts eingreifen, festgelegt wird. In Fig. 4 dieser Druckschrift ist ein alternatives Ausführungsbeispiel gezeigt, bei dem die Verriegelung des Anschlussendes des Instrumentenschafts mittels Verriegelungskugeln erfolgt.

Weitere Ansätze, die jedoch einen schaftartigen Halter mit chirurgischen Instrumenten in anderer Weise verbinden, sind in der EP 0 893 097 A2 sowie in der DE 29 09 469 B1 gezeigt. Weiterhin zeigt die EP 1 943 966 A1 eine Verbindung eines chirurgischen Einwegwerkzeuges mit einer Werkzeugaufnahme, die nur für einen einmaligen Gebrauch des Werkzeuges geeignet ist, nach Gebrauch des Werkzeuges beim Lösen desselben von der Werkzeugaufnahme zerstört wird.

Insbesondere dann, wenn - wie bei derartigen Haltern in der Regel üblich - solche Halter mit einem daran angeordneten medizinischen Instrument im Verlaufe eines operative Eingriffes eingesetzt werden, werden regelmäßig nicht nur das medizinische Instrument selbst, sondern auch der Halter, insbesondere der Bereich der Schnellkupplung verunreinigt, beispielsweise mit Blut und anderen Körperflüssigkeiten, während der Operation gelösten Gewebebestandteilen, Knochenspäne und -splittern oder dgl. Da solche Halter regelmäßig nicht für einen einmaligen Gebrauch bestimmt, sondern wieder verwendbare Gerätschaften sind, müssen sie nach entsprechendem Einsatz gereinigt und sterilisiert werden, bevor sie erneut verwendet werden können. Dabei stellen die in bekannten Schnellkupplungen vorbeschriebener Halter enthaltenen Kleinteile und insbesondere auch die gering dimensionierten Spalten und Räume zwischen derartigen Teilen bei der rückstandslosen Reinigung und vollständigen Sterilisierung regelmäßig eine besondere Herausforderung dar. Sind nämlich derartige Schnellkupplungen der Halter zerlegbar gestaltet, so müssen hier von dem für die Reinigung und Sterilisierung zuständigen Personal diese Schnellkupplungen nicht nur zerlegt, sondern die Kleinteile auch behutsam für die Reinigung und Sterilisierung gehandhabt und im Anschluss die Schnellkupplungen wieder ordnungsgemäß zusammengefügt werden. Sind hingegen die Schnellkupplungen nicht zerlegbar, so ist es häufig sogar unmöglich, kleine und kleinste Spalten und Zwischenräume, wie sie beispielsweise im Bereich des Sitzes von Verriegelungskugeln auftreten, rückstandsfrei zu reinigen und zu sterilisieren. Dort verbergen sich somit stets mögliche Gefahrenherde von Verunreinigungsrückständen, die bei einem nachfolgenden Gebrauch des Halters im Zuge eines weiteren medizinischen Eingriffes zu Komplikationen führen können.

### Darstellung der Erfindung

Es ist daher Aufgabe der vorliegenden Erfindung, einen bekannten und gattungsgemäßen Halter für ein medizinisches, insbesondere chirurgisches Instrument dahingehend weiterzubilden, dass dieser einen gegenüber herkömmlichen Haltern vereinfachten Aufbau aufweist und in der nachgebräuchlichen Nachsorge einfach zu reinigen und zu sterilisieren ist.

Diese Aufgabe wird gelöst durch einen Halter für ein medizinisches, insbesondere chirurgisches Instrument mit den Merkmalen des Patentanspruches 1. Vorteilhafte Weiterbildungen eines solchen Halters sind in den abhängigen Ansprüchen 2 bis 8 angegeben. Einen weiteren Aspekt einer Lösung der erfindungsgemäßen Aufgabe besteht sodann in einer Kombination aus einem wie angegebenen, neuartigen Halter und wenigsten einem in dem Halter lösbar festlegbaren medizinischen Instrument mit den Merkmalen des Anspruches 9 sowie entsprechende vorteilhafte Weiterbildungen gemäß der Ansprüche 10 und 11.

Erfindungsgemäß hat also ein neuartiger Halter für ein medizinisches, insbesondere chirurgisches, Instrument eine Schnellkupplung für die Aufnahme eines einen Hinterschnitt aufweisenden Anschlussendes eines Instrumentenschafts des medizinischen Instrumentes. Die Schnellkupplung weist ein erstes Kupplungselement mit einer ersten axialen Durchgangsöffnung für das Anschlussende auf. Sie weist ferner ein zweites Kupplungselement auf mit einer zweiten axialen Durchgangsöffnung für das Anschlussende. Erstes und zweites Kupplungselement sind relativ zueinander in einer Richtung quer zu der axialen Richtung der Durchgangsöffnung bewegbar aus einer Riegelstellung, in der die erste und die zweite Durchgangsöffnung dergestalt zueinander versetzt sind, dass ein Rand der zweiten Durchgangsöffnung einen Hinterschnitt an dem Anschlussende des Instrumentenschafts verriegelnd hintergreift, in eine Freigabestellung, in der die erste und die zweite axiale Durchgangsöffnung zumindest so weit in Flucht liegen, dass das Anschlussende durch beide Durchgangsöffnungen frei hindurchgeführt werden kann. Ferner sind erfindungsgemäß das erste und das zweite Kupplungselement über einer Federbrücke einstückig miteinander verbunden, und die Federbrücke spannt das erste und das zweite Kupplungselement relativ zueinander in die Riegelstellung vor.

Bei einer derartigen Ausgestaltung des Halters in seiner Schnellkupplung entfällt zunächst das Erfordernis, kleinteilige und gesonderte Riegelelemente, wie etwa Riegelkugeln, vorzusehen. Die Verriegelungswirkung wird allein durch den Versatz der beiden Durchgangsöffnungen in dem ersten und dem zweiten Kupplungselement bewirkt, den diese in der aufgrund der Vorspannung als Normalstellung eingenommenen Riegelstellung zueinander in eine Richtung quer zur axialen Erstreckung aufweisen. Mit anderen Worten ist hierbei ein mit dem Anschlussende seines Instrumentenschafts durch die erste Durchgangsöffnung hindurch geführtes medizinisches Instrument zunächst einmal durch diese Durchgangsöffnung in radialer Richtung festgelegt. Eine axiale Fixierung bzw. Verriegelung erfolgt dadurch, dass das Anschlussende auch die zweite Durchgangsöffnung durchragt, wobei die zweite Durchgangsöffnung gegenüber der ersten Durchgangsöffnung quer zur axialen Richtung derart verschoben ist, dass sie mit ihrem Rand an einem Hinterschnitt des Anschlussendes anliegt, diesen hintergreift und somit den Instrumentenschaft in axialer Richtung verriegelt. Wie oben bereits erwähnt, kann bei dieser Verriegelungsstellung weiterhin ein gewisses axiales Spiel vorhanden sein. Häufig ist ein solches axiales Spiel, jedenfalls bei handbetriebenen Haltern, von den Anwendern sogar gewünscht, da es eine zusätzliche taktile Hilfe gibt. In anderen Fällen, beispielsweise bei Instrumenten zum Durchbohren harten Knochens im Bereich der Schädeldecke, ist ein solches axiales Spiel erforderlich, da eine Drehmitnahme eines Antriebes nur in einer axial nach hinten verschobenen Stellung des Instrumentenschafts greift, die gegen eine Federlast und beim Andrücken des Instrumentes auf die zu durchbohrende Schädeldecke eingenommen wird, diese Stellung dann jedoch, wenn die Schädeldecke durchbohrt ist, entspannt, die Feder den Drehantrieb stoppt und somit das Bohrwerkzeug auskuppelt. Damit wird verhindert, dass der Bohrantrieb weiter besteht und eine Verletzung der unterhalb der Schädeldecke liegenden Weichgewebeteile oder des Gehirns erfolgt.

Der weiterhin gegebene Vorteil des erfindungsgemäßen Halters besteht darin, dass dieser in seiner Verbindung zwischen erstem und zweitem Kupplungselement einstückig gebildet ist. Auch dadurch entfällt hier eine mögliche Zerlegung in Einzelteile und Kleinteile, die beim Reinigen und Sterilisieren des Halters erforderlich wäre. Zudem können bei entsprechender und insbesondere bevorzugter Konstruktion und Ausgestaltung des Halters, insbesondere seiner Schnellkupplung, entsprechende Zwischenräume zwischen den miteinander einstückig verbundenen Elementen geschaffen sein, die sich mit einem entsprechenden Reinigungsinstrumentarium gut erreichen, reinigen und anschließend sterilisieren lassen. So können insbesondere äußerst schmale Zwischenräume vermieden werden, wie sie beispielsweise an Kugellagerflächen von Verriegelungskugeln gegeben sind und die beim Reinigen und Sterilisieren kaum vollständig und für eine gründliche Reinigung und Sterilisierung ausreichend erreichbar sind.

Die Schnellkupplung kann mit Vorteil insgesamt einstückig gebildet sein und besteht bevorzugt aus einem leicht sterilisierbaren und für den operativen Einsatz ausreichend biokompatiblen Material, wie insbesondere einem medizinisch verträglichen Edelstahl. Die Verwendung eines solchen Metalls (es kämen auch andere medizinisch verwendbare Metalle wie Titan oder Titanlegierungen in Betracht) ist auch deshalb von Vorteil, da sich mit solchem Material mit vergleichsweise dünnwandiger Bauweise eine eine ausreichende Rückstellkraft in Richtung der Riegelstellung ausbildende Federbrücke als einstückige Verbindung zwischen dem ersten und dem zweiten Kupplungselement realisieren lässt.

Der erfindungsgemäße Halter kann in seiner Schnellkupplung mit Vorteil zudem Sperrstrukturen aufweisen zum Zusammenwirken mit Gegenstrukturen an dem Anschlussende des Instrumentenschaftes für eine verdrehfeste Anordnung des Instruments in dem Halter. Eine derartige Ausgestaltung ist insbesondere dann relevant, wenn mit dem Halter Drehkräfte bzw. Drehmomente auf das medizinische Instrument, z.B. einen Bohrer oder eine Reibahle, zu übertragen sind. Eine mögliche Ausgestaltung, derartige Sperrstrukturen zu bilden, besteht darin, an der Schnellkupplung an einer außen liegenden Stirnseite des ersten Kupplungselementes auf einander gegenüberliegenden Seiten der die Oberfläche an der Stirnseite durchbrechenden Durchgangsöffnung Seitenbacken anzuordnen, die von der Oberfläche an der Stirnseite nach außen abstehen und die die Sperrstrukturen bildende, zueinander parallele und einander zugewandte ebene Anlageflächen aufweisen. Diese Anlageflächen sind dabei für die Anlage an an dem Anschlussende des Instrumentenschaftes ausgebildeten, die Gegenstrukturen bildenden Gegenflächen vorgesehen.

In einer bevorzugten Ausgestaltung des erfindungsgemäßen Halters weist die Schnellkupplung einen Grundkorpus auf, an dem das erste und das zweite Kupplungselement angeformt, insbesondere mit diesem einstückig gebildet, sind, wobei in dem Grundkorpus eine Ausnehmung vorgesehen ist, die einseitig durch eine Wand des Grundkorpus begrenzt ist und entlang derer sich auf einer der Wand gegenüberliegenden Seite die Federbrücke und zu dieser abgewinkelt das zweite Kupplungselement erstreckt. Diese Ausgestaltung ist eine besonders einfach aufgebaute, in ihrer Struktur einfach zu bildende Gestaltungsvariante, die zugleich die Erfordernisse der einfachen Reinigbarkeit und Sterilisierbarkeit des Halters insbesondere im Bereich seiner Schnellkupplung erfüllt. Denn insbesondere dann, wenn die Ausnehmung ausreichend groß dimensioniert ist, der Zwischenraum zwischen der Wand des Grundkorpus und der Federbrücke für die Einführung von Reinigungsgerätschaften hinreichend weit bemessen ist, kann dieser Abschnitt einfach gereinigt und sterilisiert werden. Dabei kann, muss indes nicht zwangsläufig, die Ausnehmung ebenfalls winkelförmig sein, dabei insbesondere in ihrem Verlauf der Winkelform des Verlaufes der Federbrücke und des abgewinkelt zu diesem verlaufenden zweiten Kupplungselementes folgen.

Ferner kann es gemäß einer vorteilhaften Ausgestaltungsvariante des erfindungsgemäßen Halters von Vorteil sein, wenn die Ausnehmung einen Rücksprung und das zweite Kupplungselement einen Vorsprung (selbstverständlich auch in umgekehrter Gestaltung, also einen Vorsprung an der Ausnehmung und einen Rücksprung an dem Kupplungselement) aufweisen, die zusammen einen eine über die Riegelstellung hinausgehende Relativbewegung der Kupplungselemente verhindernden Anschlag bilden. Ein solcher Anschlag verhindert Fehlbedienungen und erhöht insgesamt die Stabilität und Zuverlässigkeit des erfindungsgemäßen Halters.

Gemäß einer weiteren vorteilhaften Ausgestaltung der Erfindung kann die Federbrücke auf einer seitlichen Außenseite des Halters liegen und dabei derart gestaltet sein, dass auf diese manuell eine quer zu ihrer Längserstreckung und entgegen der Federwirkung der Federbrücke wirkende Kraft, insbesondere eine Druckkraft, aufgebracht werden kann, zum Verlagern des zweiten Kupplungselementes aus der Riegelstellung in die Freigabestellung. Mit anderen Worten ist die Federbrücke hier zugleich ein "Druckschalter", durch dessen Betätigung die Relativposition des ersten und des zweiten Kupplungselementes zueinander aus der Riegelstellung in die Freigabestellung überführt werden kann, um in dieser Position insbesondere ein mit dem Halter verbundenes Instrument von dem Halter zu lösen, ggf. auch ein Instrument mit den Anschlussende des Instrumentenschafts in die Schnellkupplung des Halters einzuführen und daran festzulegen.

Der Halter kann insbesondere ein manuell zu betätigender Halter sein mit einem Drehschaft, an welchen an einem freien Ende die Schnellkupplung angeordnet ist und an welchem ferner an einem dem freien Ende gegenüberliegenden Ende
T-förmig ein Griffteil angeordnet ist. Die Erfindung ist aber nicht auf einen derartigen Halter beschränkt, ein erfindungsgemäßer Halter kann auch in anders geformten manuell zu bedienenden Gestaltungsformen verwirklicht werden, wie er auch als Bestandteil eines motorisch betriebenen Instrumententriebes, sei es ein in axialer Richtung betätigter Antrieb, sei es ein rotatorisch betriebener Antrieb, verwirklicht sein kann.

In einem weiteren Aspekt der Erfindung besteht diese in einer Kombination aus einem wie oben näher beschriebenen Halter und wenigsten einem an dem Halter lösbar festlegbaren medizinischen, insbesondere chirurgischen Instrument, welches einen ein Anschlussende aufweisenden Instrumentenschaft beinhaltet und bei dem an dem Anschlussende des Instrumentenschafts ein Hinterschnitt ausgebildet ist. Dabei ist die Kombination nicht beschränkt auf einen Halter und ein einziges Instrument, es kann eine solche zugleich auch ein Set bestehend aus einem oder mehreren Haltern und einem oder mehreren medizinischen Instrumenten umfassen.

Bei einer solchen Kombination kann es von Vorteil sein, wenn, wie gemäß einer vorteilhaften Ausgestaltung der Erfindung angegeben, das Instrument an dem Anschlussende seines Instrumentenschafts eine sich von dem freien Ende des Instrumentenschafts her im weiteren, von dem freien Ende weg weisenden Verlauf konisch aufweitende Verdickung aufweist, die an ihrem dem freien Ende abgewandten Endabschnitt unter Ausbildung des Hinterschnitts zurückfällt, auf einen geringeren Umfang des Instrumentschafts. Diese konische Verdickung dient einem einfacheren Einführen des Anschlussendes des Instrumentenschafts in die Schnellkupplung, da durch den konischen Abschnitt beim Aufbringen einer in axialer Richtung gerichteten Druckkraft auf den Instrumentenschaft das zweite Kupplungselement der Schnellkupplung aus der Riegelstellung verdrängt und in die Freigabestellung bewegt wird, bis der hinter dem Konus liegende Hinterschnitt erreicht wird, der Durchmesser des Instrumentenschafts zurückfällt und das zweite Kupplungselement in die Riegelstellung schnappt.

Für eine Übertragung von Drehkräften bzw. Drehmomenten ist es von Vorteil, wenn das Instrument einer solchen Kombination an seinem Anschlussende des Instrumentenschafts die Gegenflächen bildende, parallele Abflachungen aufweist.

### Kurze Beschreibung der Zeichnungen

Weitere Vorteile und Merkmale der Erfindung ergeben sich aus der nachfolgenden Beschreibung eines Ausführungsbeispiels anhand der beigefügten Figuren. Dabei zeigen:

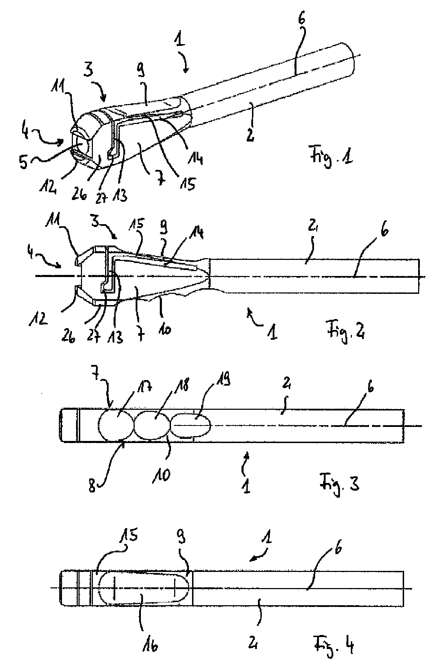

Fig. 1 ein Ausführungsbeispiel eines erfindungsgemäßen Halters für ein medizinisches, insbesondere chirurgisches Instrument in einer perspektivischen Darstellung schräg von einer die Schnellkupplung aufweisenden Seite her;
Fig. 2 den Halter aus Fig. 1 in einer Seitenansicht mit Blick auf eine Seitenfläche der Schnellkupplung;
Fig. 3 den Halter aus Fig. 1 in einer Seitendarstellung mit Blick auf eine erste Schmalseite;
Fig. 4 den Halter aus Fig. 1 in einer Seitendarstellung mit Blick auf eine weitere, der in Fig. 3 gezeigten Schmalseite gegenüberliegenden Schmalseite;
Fig. 5 eine Vorderansicht des Halters gemäß Fig. 1 von der Kupplungsseite der Schnellkupplung her in vergrößerter Darstellung;
Fig. 6 eine Längsschnittdarstellung durch den Halter gemäß Fig. 1;
Fig. 7 den Halter gemäß Fig. 1 mit darin aufgenommenem Anschlussende eines medizinischen Instrumentes in teilgeschnittener Darstellung; und
Fig. 8 in drei Darstellungen a bis c vergrößert den Abschnitt des Halters gemäß Fig. 1 mit der Schnellkupplung beim Einführen eines Anschlussendes eines medizinischen Instrumentes zum Festlegen desselben an dem Halter.

In den Figuren ist ein beispielhaftes Ausführungsbeispiel eines erfindungsgemäßen Halters gezeigt und wird nachstehend näher erläutert. Die Figuren sind dabei nicht als vollständige Konstruktionszeichnungen anzusehen, sie beschränken sich vielmehr auf die Darstellung der erfindungswesentlichen und weiterer relevanter Merkmale des Ausführungsbeispiels eines erfindungsgemäßen Halters.

Ein erfindungsgemäßer Halter für ein medizinisches, insbesondere chirurgisches Instrument ist in den Figuren in verschiedenen (Teil-) Ansichten gezeigt und allgemein mit dem Bezugszeichen 1 bezeichnet.

### Weg(e) zur Ausführung der Erfindung

Der Halter 1 in diesem Ausführungsbeispiel ist langgestreckt geformt mit einem Schaftabschnitt 2. An einem stirnseitigen Ende dieses Schaftabschnittes 2 ist eine Schnellkupplung 3 angeformt, die der Aufnahme eines einen Hinterschnitt aufweisenden Anschlussendes eines Instrumentenschaftes des medizinischen Instrumentes, mit welchem der Halter zu verbinden ist, dient.

Die Schnellkupplung 3 weist einen Hauptkorpus 26 und an dessen Stirnseite 4 eine Aufnahmeöffnung 5 auf, die die Form einer entlang einer Längsachse 6 des Halters 1 geführten Bohrung hat. Die Schnellkupplung 3 ist insbesondere einstückig gebildet und weist zwei abgeflachte Seitenflächen 7 und 8 auf sowie zwischen diesen verlaufende Schmalseiten 9 und 10, die sich keilförmig öffnend auseinander verlaufen zu einem der Stirnseite 4 nahegelegenen Abschnitt, von wo aus sich die Schmalseite 9, 10 wieder verjüngen. Dort gehen die Schmalseiten 9, 10 in zwei an der Stirnseite 4 vorstehende Vorsprünge 11 bzw. 12 über.

Eine Kupplungsplatte 13 ist durch entsprechende Ausschnitte aus dem einstückigen Material der Schnellkupplung 3 herausgeformt und einseitig mit einer durch einen Schlitz 14 von dem Hauptkorpus 26 der Schnellkupplung 3 getrennten Federbrücke 15 verbunden. In einem der Stirnseite 4 axial gegenüberliegenden Bereich, in dem der Schlitz 14 endet, ist die Federbrücke 15 mit dem Hauptkorpus 26 der Schnellkupplung 3 einstückig verbunden. Die Federbrücke 15 weist eine Abflachung 16 auf ihrer an der Schmalseite 9 gelegenen Oberfläche auf, welche Abflachung als ergonomisches bzw. haptisches Element für die Anlage des Daumens einer Hand ausgebildet ist. Auf der gegenüberliegenden Schmalseite 10 sind Griffmulden 17, 18, 19 ausgebildet. Diese dienen der Anlage von Fingern, insbesondere Zeigefinger, Mittelfinger und Ringfinger der Hand, deren Daumen in der Abflachung 16 ruht. So kann mit einer Hand die Federzunge 14 in Richtung der gegenüberliegenden Schmalseite 10 ausgelenkt werden, um damit eine Längsverschiebung der Kupplungsplatte 13 in selbiger Richtung zu bewirken. Kupplungsplatte 13 und Federbrücke 15 sind, wie bereits erwähnt, einstückig miteinander verbunden und schließen einen Winkel ein, so dass die Kupplungsplatte 13 im Wesentlichen senkrecht zu der Längsachse 6 und mithin der Richtung der als entlang dieser Achse geführten Bohrung der Aufnahmeöffnung 5 verläut. Die Kupplungsplatte 13 liegt in einem Aufnahmeschlitz 27, der in der Schnellkupplung 3, genauer in deren Hauptkorpus 26, ausgeformt ist, und in einem Winkel zu dem Schlitz 14 verläuft. In dem Aufnahmeschlitz 27 kann sich die Kupplungsplatte 13 in einer Längsrichtung bewegen. Der Aufnahmeschlitz 27 und der Schlitz 14 liegen in einem Winkel zueinander und bilden gemeinsam einen etwa L-förmigen Schlitz.

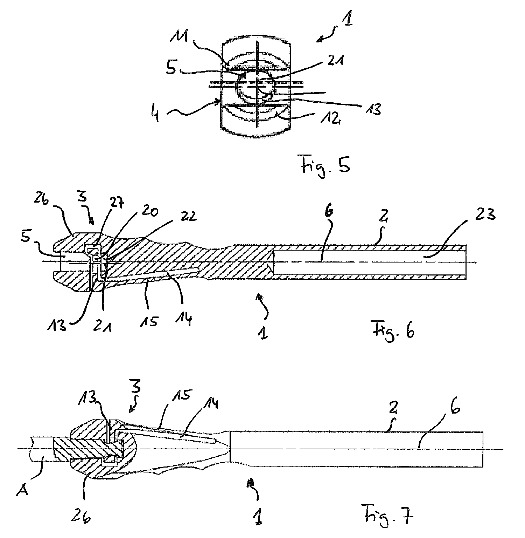

In der Kupplungsplatte 13 ist, wie insbesondere in der Fig. 6 zu erkennen ist, eine Durchtrittsöffnung 20 ausgebildet. Diese Durchtrittsöffnung 20 verläuft in ihrer Ausrichtung ebenfalls in axialer Richtung, d.h. mit einer Erstreckungsrichtung parallel zu der Längsachse 6, ist jedoch in der in Fig. 6 gezeigten Position, in der die Federbrücke 15 in einer Ausgangs- bzw. Ruheposition zu erkennen ist, mit ihrer Mittelachse 21 relativ zu der Längsachse 6, die zugleich die Mittelachse der Aufnahmeöffnung 5 bildet, versetzt. Die Aufnahmeöffnung 5 erstreckt sich über den Abschnitt, in dem die Kupplungsplatte 13 diese quert, hinaus bis zu einem Endabschnitt 22. Zu erkennen ist dies auch noch einmal in der Vorderansicht gemäß Fig. 5, in welcher die versetzten Achsen Mittelachse 21 und Längsachse 6 gezeigt sind sowie ein bei Ansicht auf die Aufnahmeöffnung 5 durch diese hindurch zu erkennender Abschnitt der Kupplungsplatte 13.

In Fig. 6 ist zudem zu sehen, dass in dem Schaftabschnitt 2 des Halters 1 eine als Sackloch ausgebildete Aufnahme 23 geformt ist, die zum Verbinden des erfindungsgemäßen Halters 1 mit weiteren Strukturen, beispielsweise der Antriebswelle eines motorischen Instrumentenantriebes mit einem rotatorischen Antrieb oder aber mit einem, beispielsweise als quer zu der Längserstreckung des Halters 1, also quer zu der Längsachse 6, insbesondere senkrecht zu dieser, ausgebildeten T-Griff dient.

Die Schnellkupplung 3 des erfindungsgemäßen Halters 1 ist zur Verbindung mit einem Anschlussende A (vgl. Fig. 7) eines Instrumentenschaftes eingerichtet. In der hier gezeigten Konstruktion und Gestaltung ist die Schnellkupplung 3 bzw. das Anschlussende A insbesondere als sogenannter Hudson-Anschluss bzw. eine entsprechende Anschlusskupplung gebildet.

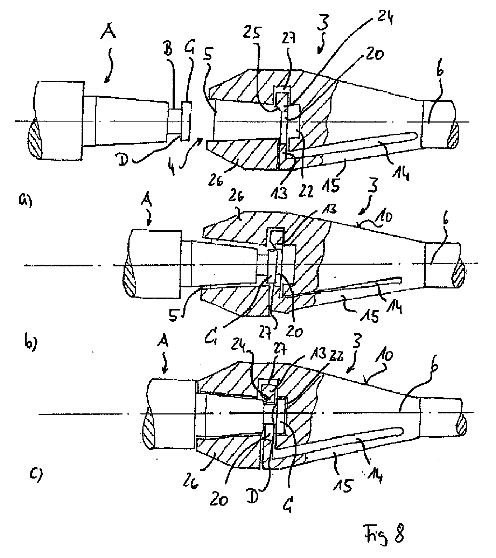

Die Vorgehensweise beim Verbinden des Anschlussendes A mit der Schnellkupplung 3 des Halters 1 ist in den drei Darstellungen und Skizzen der Fig. 8 noch einmal gesondert dargestellt und wird nachfolgend anhand dieser Darstellungen beschrieben.

In einem ersten, in der Fig. 8a dargestellten Ausgangszustand ist die Schnellkupplung 3 des Halters mit der Federbrücke 15 in einer entspannten Ausgangs- bzw. Ruhestellung gezeigt. In dieser Stellung sind die Aufnahmeöffnung 5 und die Durchtrittsöffnung 20 in der Kupplungsplatte 13 nicht in exakter Flucht ausgerichtet. Vielmehr ragt ein die Durchtrittsöffnung 20 begrenzender Abschnitt 24 der Kupplungsplatte 13 in den Bereich der Aufnahmeöffnung 5 hinein und überdeckt insoweit insbesondere deren Endabschnitt 22. In dem Abschnitt 24 ist, wie in dieser vergrößerten Darstellung zu erkennen ist, die Kupplungsplatte 13 mit einer der Stirnseite 4 zugewandten Anlaufschräge 25 versehen.

Das Anschlussende A des Instrumentenschaftes hat an seinem freien Ende einen Bereich B verjüngten Durchmessers und einen diesen überdeckenden Abschluss C. An diesem ist ein Hinterschnitt D gebildet. Wird nun, wie in Fig. 8b gezeigt, das Anschlussende A in die Aufnahmeöffnung 5 eingeführt, so stößt der Abschluss C an die Anlaufschräge 25 der Kupplungsplatte 13. Dadurch wird diese in der figürlichen Darstellung nach oben, also in Richtung der Schmalseite 10 gedrückt. Aufgrund der Elastizität und Flexibilität der Federbrücke 15 weicht die Kupplungsplatte 13 in diese Richtung aus, so dass die Durchtrittsöffnung 20 von dem Abschluss C passiert werden kann. Genügt die Wirkung der Anlaufschräge 25 im Zusammenspiel mit dem Abschluss C nicht, um die Kupplungsplatte 13 so weit in Richtung der Schmalseite 10 ausweichen zu lassen, dass die Durchtrittsöffnung 20 für den Abschluss C passierbar ist, so kann hier von Hand nachgeholfen werden, indem die Federbrücke 15 unter Schließen des Schlitzes 14 in Richtung der gegenüberliegenden Schmalseite 10 eingedrückt wird.

Hat der Abschluss C die Durchtrittsöffnung 20 in der Kupplungsplatte 13 vollständig passiert, gelangt er in den Endabschnitt 22 der Aufnahmeöffnung 5, und die Kupplungsplatte 13 kann, getrieben durch die Federkraft der Federbrücke 15, zurückschnappen in ihre Ruhe- bzw. Ausgangsposition. In dieser Position verriegelt der Abschnitt 24, der über den Hinterschnitt D an dem Anschlussende A liegt, das Anschlussende A in axialer Richtung, d.h. in Richtung der Längsachse 6 des Halters. Um das Anschlussende A aus dieser Position zu lösen, muss die Federbrücke 15 in Richtung der gegenüberliegenden Schmalseite 10 eingedrückt, dadurch die Kupplungsplatte 13 so verschoben werden, dass die Durchtrittsöffnung 20 für einen Durchgang des Abschlusses C frei liegt und das Anschlussende A entnommen werden kann.

Aus der obigen Beschreibung des Ausführungsbeispiels ist deutlich geworden, dass die Schnellkupplung 6 des erfindungsgemäßen Halters einfach aufgebaut ist durch das Zusammenwirken von Durchgangsöffnung 5 und Durchtrittsöffnung 20 im Zusammenspiel mit der Federbrücke 15 und der entsprechenden Riegelfunktion, ferner durch die Einstückigkeit. Dadurch ergibt sich neben einer einfachen und robusten Bedienbarkeit insbesondere eine einfache Reinigbarkeit und Sterilisierbarkeit nach erfolgtem Gebrauch. Das gezeigte Ausführungsbeispiel beschränkt die Erfindung nicht, die in den nachstehenden Patentansprüchen in ihrer breiten Tragweite bestimmt ist.

### Bezugszeichenliste

- 1: Halter
- 2: Schaftabschnitt
- 3: Schnellkupplung
- 4: Stirnseite
- 5: Aufnahmeöffnung
- 6: Längsachse
- 7: Seitenfläche
- 8: Seitenfläche
- 9: Schmalseite
- 10: Schmalseite
- 11: Vorsprung
- 12: Vorsprung
- 13: Kupplungsplatte
- 14: Schlitz
- 15: Federbrücke
- 16: Abflachung
- 17: Griffmulde
- 18: Griffmulde
- 19: Griffmulde
- 20: Durchtrittsöffnung
- 21: Mittelachse
- 22: Endabschnitt
- 23: Aufnahme
- 24: Abschnitt
- 25: Anlaufschräge
- 26: Hauptkorpus
- 27: Aufnahmeschlitz
- A: Anschlussende
- B: Bereich
- C: Abschluss
- D: Hinterschnitt

## Patentansprüche

1. Halter für ein medizinisches, insbesondere chirurgisches Instrument mit einer Schnellkupplung (3) für die Aufnahme eines einen Hinterschnitt (D) aufweisenden Anschlussendes (A) eines Instrumentenschafts des medizinischen Instruments, wobei die Schnellkupplung (3) ein erstes Kupplungselement (26) mit einer ersten axialen Durchgangsöffnung (5) für das Anschlussende (A) aufweist, wobei die Schnellkupplung (3) ein zweites Kupplungselement (13) mit einer zweiten axialen Durchgangsöffnung (20) für das Anschlussende (A) aufweist, welches zweite Kupplungselement (13) relativ zu dem ersten Kupplungselement (26) in einer Richtung quer zu der axialen Richtung (6) der Durchgangsöffnungen (5, 20) aus einer Riegelstellung, in der die erste und die zweite Durchgangsöffnung (5, 20) in solcher Weise relativ zu einander versetzt sind, dass ein Rand (24) der zweiten Durchgangsöffnung (20) den Hinterschnitt (D) an dem Anschlussende (A) des Instrumentenschafts verriegelnd hintergreift, in eine Freigabestellung, in der die erste (5) und die zweite (20) axiale Durchgangsöffnung zumindest so weit in Flucht liegen, dass das Anschlussende (A) durch beide Durchgangsöffnungen (5, 20) frei hindurchgeführt werden kann, bewegbar ist, **dadurch gekennzeichnet, dass** das erste (26) und das zweite (13) Kupplungselement über eine Federbrücke (15) einstückig miteinander verbunden sind und die Federbrücke (15) das erste (26) und das zweite (13) Kupplungselement relativ zueinander in die Riegelstellung vorspannt.

2. Halter nach Anspruch 1, **dadurch gekennzeichnet, dass** die Schnellkupplung (3) Sperrstrukturen (11, 12) aufweist zum Zusammenwirken mit Gegenstrukturen an dem Anschlussende (A) des Instrumentenschaftes für eine verdrehfeste Anordnung des Instruments in dem Halter (1).

3. Halter nach Anspruch 2, **dadurch gekennzeichnet, dass** die Schnellkupplung (3) an einer außen liegenden Stirnseite (4) des ersten Kupplungselementes (26) auf einander gegenüberliegenden Seiten der die Oberfläche an der Stirnseite (4) durchbrechenden Durchgangsöffnung (5) angeordnete von der Oberfläche an der Stirnseite (4) nach außen abstehende Seitenbacken (11, 12) aufweist mit die Sperrstrukturen bildenden zueinander parallelen, einander zugewandten ebenen Anlageflächen für die Anlage an an dem Anschlussende (A) des Instrumentenschaftes ausgebildeten, die Gegenstrukturen bildenden Gegenflächen.

4. Halter nach einem der vorhergehenden Ansprüche, **dadurch gekennzeichnet, dass** die Schnellkupplung (3) einen Grundkorpus aufweist, an dem das erste (26) und das zweite (13) Kupplungselement angeformt sind, wobei in dem Grundkorpus eine Ausnehmung (14, 27) vorgesehen ist, die einseitig durch eine Wand des Grundkorpus begrenzt ist und entlang derer sich auf einer der Wand gegenüberliegenden Seite die Federbrücke (15) und zu dieser abgewinkelt das zweite Kupplungselement (13) erstreckt.

5. Halter nach Anspruch 4, **dadurch gekennzeichnet, dass** die Ausnehmung (14, 27) winkelförmig ist.

6. Halter nach einem der Ansprüche 3 oder 4, **dadurch gekennzeichnet, dass** die Ausnehmung (14, 27) einen Rücksprung und das zweite Kupplungselement (13) einen Vorsprung aufweisen, die zusammen einen eine über die Riegelstellung hinausgehende Relativbewegung der Kupplungselemente (13) verhindernden Anschlag bilden.

7. Halter nach einem der vorhergehenden Ansprüche, **dadurch gekennzeichnet, dass** die Federbrücke (15) auf einer seitlichen Außenseite (9) des Halters (1) liegt und derart gestaltet ist, dass auf diese manuell eine quer zu ihrer Längserstreckung und entgegen der Federwirkung der Federbrücke (15) wirkende Kraft, insbesondere Druckkraft, aufgebracht werden kann zum Verlagern des zweiten Kupplungselementes (13) aus der Riegelstellung in die Freigabestellung.

8. Halter nach einem der vorhergehenden Ansprüche, **dadurch gekennzeichnet, dass** er einen Drehschaft (2) aufweist, an welchem an einem freien Ende die Schnellkupplung (3) angeordnet ist und an welchem an einem gegenüberliegenden Ende T-förmig ein Griffteil angeordnet ist.

9. Kombination bestehend aus einem Halter (1) nach einem der vorherigen Ansprüche und wenigstens einem an dem Halter (1) lösbar festlegbaren medizinischen, insbesondere chirurgischen Instrument mit einem ein Anschlussende (A) aufweisenden Instrumentenschaft, wobei an dem Anschlussende (A) des Instrumentenschaftes ein Hinterschnitt (D) ausgebildet ist.

10. Kombination nach Anspruch 9, **dadurch gekennzeichnet, dass** das Instrument an dem Anschlussende (A) seines Instrumentenschafts eine sich von dem freien Ende des Instrumentenschafts her im weiteren, von dem freien Ende weg weisenden Verlauf konisch aufweitende Verdickung aufweist, die an ihrem dem freien Ende abgewandten Endabschnitt unter Ausbildung des Hinterschnitts (D) zurückfällt auf einen geringeren Umfang des Instrumentenschafts.

11. Kombination nach einem der Ansprüche 9 oder 10, **dadurch gekennzeichnet, dass** an dem Anschlussende (A) des Instrumentenschafts Gegenflächen bildende, parallele Abflachungen ausgebildet sind.

## Claims

1. Holder for a medical instrument, in particular a surgical instrument, with a quick coupling (3) for receiving a connecting end (A) of an instrument shaft of the medical instrument, said connecting end (A) having an undercut (D), wherein the quick coupling (3) has a first coupling element (26) with a first axial through-opening (5) for the connecting end (A), wherein the quick coupling (3) has a second coupling element (13) with a second axial through-opening (20) for the connecting end (A), which second coupling element (13) is movable relative to the first coupling element (26) in a direction transverse to the axial direction (6) of the through-openings (5, 20) from a locking position, in which the first and second through-openings (5, 20) are offset relative to each other in such a way that an edge (24) of the second through-opening (20) engages lockingly behind the undercut (D) on the connecting end (A) of the instrument shaft, to a release position, in which the first axial through-opening (5) and the second axial through-opening (20) are aligned at least to such an extent that the connecting end (A) can be guided freely through both through-openings (5, 20), **characterized in that** the first coupling element (26) and the second coupling element (13) are connected integrally to each other via a spring bridge (15), and the spring bridge (15) pre-stresses the first coupling element (26) and the second coupling element (13) into the locking position relative to each other.

2. Holder according to Claim 1, **characterized in that** the quick coupling (3) has blocking structures (11, 12) for interaction with mating structures on the connecting end (A) of the instrument shaft for a rotationally fixed arrangement of the instrument in the holder (1).

3. Holder according to Claim 2, **characterized in that**, on an outer front face (4) of the first coupling element (26), the quick coupling (3) has side jaws (11, 12) which are arranged on mutually opposite sides of the through-opening (5) passing through the surface on the front face (4) and which protrude outwards from the surface on the front face (4), with mutually parallel planar contact surfaces facing one another and forming the blocking structures, for bearing on mating surfaces provided on the connecting end (A) of the instrument shaft and forming the mating structures.

4. Holder according to one of the preceding claims, **characterized in that** the quick coupling (3) has a main body on which the first coupling element (26) and the second coupling element (13) are integrally formed, wherein a recess (14, 27) is provided in the main body and is delimited on one side by a wall of the main body and along which the spring bridge (15) extends on a side opposite the wall, while the second coupling element (13) extends at an angle to the spring bridge (15).

5. Holder according to Claim 4, **characterized in that** the recess (14, 27) has an angled shape.

6. Holder according to either of Claims 3 and 4, **characterized in that** the recess (14, 27) has a setback, and the second coupling element (13) has a protrusion, said setback and protrusion together forming a stop which prevents a relative movement of the coupling elements (13) beyond the locking position.

7. Holder according to one of the preceding claims, **characterized in that** the spring bridge (15) lies on a lateral outer face (9) of the holder (1) and is configured in such a way that a force, in particular a pressure force, acting transversely with respect to its longitudinal extent and counter to the spring action of the spring bridge (15) can be applied manually in order to displace the second coupling element (13) from the locking position to the release position.

8. Holder according to one of the preceding claims, **characterized in that** it has a rotary shaft (2) on which the quick coupling (3) is arranged at one free end and on which a grip part is arranged in a T shape at an opposite end.

9. Combination consisting of a holder (1) according to one of the preceding claims and of at least one medical instrument, in particular a surgical instrument, which can be fastened releasably to the holder (1), said instrument having an instrument shaft with a connecting end (A), wherein an undercut (D) is provided on the connecting end (A) of the instrument shaft.

10. Combination according to Claim 9, **characterized in that**, at the connecting end (A) of its instrument shaft, the instrument has a conically widening thickened area extending from the free end of the instrument shaft, in the remaining course pointing away from the free end, which thickened area, at its end portion directed away from the free end, falls back to a smaller circumference of the instrument shaft to form the undercut (D).

11. Combination according to either of Claims 9 and 10, **characterized in that** parallel flattened areas that form mating surfaces are provided on the connecting end (A) of the instrument shaft.

## Revendications

1. Support pour un instrument médical, en particulier chirurgical avec un couplage rapide (3) destiné à recevoir une extrémité de raccordement (A) d'une tige d'instrument de l'instrument médical, qui présente une contre-dépouille (D), dans lequel le couplage rapide (3) présente un premier élément de couplage (26) avec une première ouverture de passage axiale (5) pour l'extrémité de raccordement (A), dans lequel le raccord rapide (3) présente un deuxième élément de couplage (13) avec une deuxième ouverture de passage axiale (20) pour l'extrémité de raccordement (A), lequel deuxième élément de couplage (13) est déplaçable par rapport au premier élément de couplage (26) dans une direction transversale à la direction axiale (6) des ouvertures de passage (5, 20), d'une position de verrouillage dans laquelle la première et la deuxième ouvertures de passage (5, 20) sont décalées l'une par rapport à l'autre de telle manière qu'un bord (24) de la deuxième ouverture de passage (20) s'accroche en position verrouillée à la contre-dépouille (D) sur l'extrémité de raccordement (A) de la tige d'instrument, à une position de libération dans laquelle la première (5) et la deuxième (20) ouvertures de passage axiales sont en alignement, au moins dans une mesure telle que l'extrémité de raccordement (A) puisse être introduite librement à travers les deux ouvertures de passage (5, 20), **caractérisé en ce que** le premier (26) et le deuxième (13) éléments de couplage sont reliés l'un à l'autre en une seule pièce par un pont à ressort (15) et le pont à ressort (15) précontraint le premier (26) et le deuxième (13) éléments de couplage l'un par rapport à l'autre dans la position de verrouillage.

2. Support selon la revendication 1, **caractérisé en ce que** le raccord rapide (3) présente des structures d'arrêt (11, 12) pour coopérer avec des structures opposées sur l'extrémité de raccordement (A) de la tige d'instrument pour un agencement sans rotation de l'instrument dans le support (1).

3. Support selon la revendication 2, **caractérisé en ce que** le raccord rapide (3) présente sur un côté frontal situé extérieurement (4) du premier élément de couplage (26) des mâchoires latérales (11, 12) sur des côtés opposés l'un à l'autre de l'ouverture de passage (5) traversant la surface sur le côté frontal (4) et saillantes vers l'extérieur sur la surface du côté frontal (4), avec des faces d'appui planes tournées l'une vers l'autre, parallèles l'une à l'autre et formant les structures d'arrêt, pour l'appui sur des faces opposées formées sur l'extrémité de raccordement (A) de la tige d'instrument et formant les structures opposées.

4. Support selon l'une quelconque des revendications précédentes, **caractérisé en ce que** le raccord rapide (3) présente un corps de base, sur lequel le premier (26) et le deuxième (13) éléments de couplage sont formés, dans lequel il est prévu dans le corps de base un évidement (14, 27), qui est limité d'un côté par une paroi du corps de base et le long duquel le pont à ressort (15) et coudé par rapport à celui-ci le deuxième élément de couplage (13) s'étend sur un côté opposé à la paroi.

5. Support selon la revendication 4, **caractérisé en ce que** l'évidement (14, 27) est en forme de cornière.

6. Support selon l'une quelconque des revendications 3 ou 4, **caractérisé en ce que** l'évidement (14, 27) présente un retrait et le deuxième élément de couplage (13) présente une saillie, qui forment ensemble une butée empêchant un mouvement relatif des éléments de couplage (13) au-delà de la position de verrouillage.

7. Support selon l'une quelconque des revendications précédentes, **caractérisé en ce que** le pont à ressort (15) est situé sur un côté extérieur latéral (9) du support (1) et est configuré de telle manière qu'une force, en particulier une force de pression, agissant transversalement à son extension longitudinale et opposée à l'action de ressort du pont à ressort (15) puisse lui être appliquée manuellement en vue du déplacement du deuxième élément de couplage (13) de la position de verrouillage à la position de libération.

8. Support selon l'une quelconque des revendications précédentes, **caractérisé en ce qu'**il présente un arbre rotatif (2), sur lequel le raccord rapide (3) est disposé à une extrémité libre et sur lequel une partie de prise est disposée en forme T à une extrémité opposée.

9. Combinaison composée d'un support (1) selon l'une quelconque des revendications précédentes et d'au moins un instrument médical, en particulier chirurgical, avec un arbre d'instrument présentant une extrémité de raccordement (A), pouvant être fixé de façon séparable, dans lequel une contre-dépouille (D) est formée à l'extrémité de raccordement (A) de l'arbre d'instrument.

10. Combinaison selon la revendication 9, **caractérisée en ce que** l'instrument présente à l'extrémité de raccordement (A) de son arbre d'instrument une surépaisseur s'évasant en forme de cône depuis l'extrémité libre de l'arbre d'instrument dans son tracé s'éloignant de l'extrémité libre, qui, à sa partie d'extrémité détournée de l'extrémité libre, retombe à un plus petit pourtour de l'arbre d'instrument en formant la contre-dépouille (D).

11. Combinaison selon une des revendications 9 ou 10, **caractérisée en ce que** des méplats parallèles formant des faces opposées sont réalisés à l'extrémité de raccordement (A) de l'arbre d'instrument.
